# EUROPEAN PATENT APPLICATION

(11) **EP 1 911 737 A1**
(43) Date of publication of application: **16.04.2008**
(21) Application number: 07016561.8
(22) Date of filing: 23.08.2007
(51) Int. Cl.: C07C 51/215

(54) **Process for preparing an organic acid or its derivatives using a homogeneous MC-Type catalyst an O2/CO2 mixture**

(30) Priority: 12.10.2006 US 851052 P; 11.06.2007 KR 20070056781; 25.06.2007 KR 20070062496; 06.08.2007 KR 20070078542
(71) Applicant: Kocat Inc., Seoul 150-801 (KR)
(72) Inventor: Yoo, Jin S., Flossmoor IL 60422 (US); Lee, Sang-Chul, Asan-si 336-756 Chungnam (KR); Kim, Ho Dong, Gangneung-si Gangwon-do 210-733 (KR)
(74) Representative: Winter, Brandl, Fürniss, Hübner Röss, Kaiser, Polte Partnerschaft Patent- und Rechtsanwaltskanzlei

(57) **Abstract**

The present invention relates to a process for producing an organic acid or its derivatives, which comprises oxidation of a hydrocarbon substrate in the presence of a homogeneous MC-type catalyst and an oxidant. In particular, the present invention relates to a process comprising oxidation of a hydrocarbon substrate in the presence of a homogeneous MC-type catalyst selected from Co/Br, Mn/Br, Co/Mn/Br and Co/Mn/M/Br and an O₂/CO₂ mixed gas oxidant where the partial pressure of O₂ is controlled to 30-40%, thereby improving the oxidation rate, the conversion, the selectivity and the yield even under milder conditions. Especially, the present invention remarkably reduces down to a negligible level the production of color impurities and other impurities such as 4-CBA and p-toluic acid, which had been conventionally removed by hydrogenation reduction. Further, the loss in acetic acid solvent due to combustion can also be drastically decreased using the method of the present invention.

## Description

### CROSS REFERENCE TO RELATED APPLICATIONS

This application claims priority from U.S. provisional application No. 60/851,052 filed on October 12, 2006, and Korean patent application Nos. 10-2007-56781, 10-2007-0062496 and 10-2007-78542 filed on June 11, 2007 June 25, 2007 and August 6, 2007, respectively, all of which are incorporated herein by reference in its entirety for all purposes.

### TECHNICAL FIELD

The present invention relates to a process for producing an organic acid or its derivatives, which comprises oxidation of a hydrocarbon substrate in the presence of a homogeneous MC-type catalyst and an oxidant. In particular, the present invention relates to a process comprising oxidation of a hydrocarbon substrate in the presence of a homogeneous MC-type catalyst selected from Co/Br, Mn/Br, Co/Mn/Br and Co/Mn/M/Br, and an O₂/CO₂ mixed gas oxidant where the partial pressure of O₂ is controlled to 30-40% of the mixed gas, thereby improving the oxidation rate, the conversion, the selectivity and the yield even under milder conditions. Especially, the present invention remarkably reduces down to a negligible level the production of color impurities and other impurities such as 4-carboxybenzaldehyde (referred to as "4-CBA" hereinafter) and p-toluic acid, which had been conventionally removed by hydrogenation reduction. Further, the loss in acetic acid solvent due to combustion can also be drastically decreased by using the method of the present invention.

### RELATED PRIOR ART

As discussed below, methods of manufacturing aromatic carboxylic acids have been well known and widely used commercially. For example, the method of manufacturing of aromatic carboxylic acids such as terephthalic acid (referred to as "TA" hereinafter), isophthalic acid ("IPA"), phthalic acid, phthalic anhydride, naphthalene dicarboxylic acid, trimellitic acid, trimellitic anhydride, trimesic acid, pyromellitic dianhydride, 4,4'-biphenyldicarboxylic acid and benzoic acid by oxidizing alkylaromatic compounds or their oxidized intermediates thereof, in the presence of cobalt-manganese-bromine, from such alkylaromatic compounds as *p*-xylene, *p*-tolualdehyde, *p*-toluic acid, 4-CBA, meta-xylene, meta-tolualdehyde, meta-toluic acid, 3-carboxybenzaldehyde, ortho-xylene, dimethylnaphtalene, pseudocumene (1,2,4-trimethylbenzene), mesitylene (1,3,5-trimethylbenzene), durene (1,2,4,5-tetramethylbenzene), 4,4'-dimethylbiphenyl and toluene is well known (for example, U.S. patent Nos. 2,833,816 and 5,183,933). Su ch aromatic carboxylic acids have been used as raw materials for manufacturing polyester after appropriate purification such as hydrogenation, etc. (U.S. patent No. 3,584,039). Also, polyester has been widely used as a synthetic fiber, a film, etc.

Many efforts have been made to develop a catalyst system with high efficiency and enhanced reactivity to manufacture aromatic carboxylic acids. The newly developed technologies, however, are not practical due to the increase in side reactions, high price of catalysts, difficulty in operation, precipitation of catalysts, etc.

Improving reaction efficiency in manufacture of aromatic carboxylic acids can greatly reduce reaction time and side reactions thereby improving productivity, quality and cost effectiveness. Therefore, it is highly desirable to develop a technology to increase the efficiency of the oxidation reaction of alkyl aromatic compounds and the oxidized intermediates thereof by means of an improvement in the reaction processes.

There have been also various efforts to increase the efficiency by adding a third metal catalyst to the cobalt-manganese-bromine catalyst system, the basic catalyst system, to enhance the catalyst efficiency during the manufacture of aromatic carboxylic acids. The added metals were mainly transition metals, and by adding, for example, hafnium, zirconium, molybdenum, etc., the reactivity was increased (U.S. patent No. 5,112,992).

On the other hand, an oxygen containing gas such as air was mainly used as an oxidant during the manufacture of aromatic carboxylic acids. Carbon dioxide was not used as an oxidant because it is chemically stable. Yet, in an effort to improve the process efficiency, there was a case in which chemically stable carbon dioxide, recycled from the reaction vent gas, was injected to a reactor to increase the stability in the process by mitigating the problematic possibilities of explosion due to oxygen when using pure oxygen or a gas containing pure oxygen or oxygen enriched gas as an oxidant (U.S. patent No. 5,693,856). Nevert heless, there has not been known any case where carbon dioxide was added to improve the reaction efficiency and the effects of the concentration of added carbon dioxide on oxidation.

The current commercial processes for preparing purified terephthalic acid (referred to as "PTA" hereinafter) comprise two main steps: (i) synthesis of crude terephthalic acid (referred to as "CTA" hereinafter) and (ii) purification of the CTA by hydrogenation.

The CTA produced by the commercial processes contains various impurities as shown in TABLE 1. In particular, 4-CBA and p-toluic acid show an adverse effect on the production of poly(ethylene terephthalate) ("PET") because they act as chain terminators in the condensation reaction between PTA and ethylene glycol. Further, the color impurities such as 2,6-dicarboxyanthraquinone (referred to as "2,6-DCAq" hereinafter) have serious adverse effects on the color of PET products. Thus, the CTA produced in the commercial process should undergo post-treatment of hydrogenation using Pd/C catalyst to remove these impurities.

**TABLE 1**

| | |
|---|---|
| 4-CBA | ~ 3000 ppm |
| *p*-Toluic-acid | ~ 500 ppm |
| Color impurities* | ~ 300 ppm |
| Metal impurities* | ~ 100 ppm |
| Water and ash | ~ 100 ppm |
| * Color impurities: benzyl, fluorenone and anthraquinone | |
| * Metal impurities: Fe and other metals such as Co and Mn derived from catalyst | |

Samsung General Chemical Co., Ltd. disclosed an invention, where carbon dioxide (nitrogen included) is added in air, which has conventionally been used as an oxidant, and co-catalysts such as K and Zr are also introduced [U.S. patent Nos. 6,194,607 and 6,180,822; Japanese patent No. 2002-543172].

Korean Research Institute of Chemical Technology disclosed a process, where CO₂-added air or a premixed gas of O₂ and CO₂ was used as an oxidant and a co-catalyst such as Fe and Ni was also introduced [Korean patent application publication Nos. 10-2006-00611134 and 10-2005-0068565; Korean patent Nos. 10-050868 and 10-0427298]. However, they only disclosed the partial pressure of O₂ of lower than 26%, and failed to suggest synergism caused by the partial pressure of O₂ of higher than 30%.

Further, these inventions have serious problems that products contain a relatively large amount of various impurities including 4-CBA and p-toluic acid, particularly color impurities including benzyl, fluorenone and anthraquinone. Thus, they still require a complicated and high-cost purification process as in other conventional processes.

To overcome the aforementioned problems, the present invention aims to provide a process for preparing an organic acid or its derivatives, which may be applied to the conventional reactors without modification. The aimed process herein shows the following advantages by using high highly concentrated O₂ and maximizing the reaction conditions: (i) the production of various impurities such as 4-CBA, p-toluic acid as well as color impurities such as benzyl, fluorenone and anthraquinone can be much reduced so that no purification is required, (ii) the oxidation rate, the conversion, the selectivity and the yield are improved even under milder conditions, and (iii) the loss in acetic acid solvent due to combustion can be drastically decreased.

### DETAILED DESCRIPTION

The present invention relates to a process for preparing an organic acid or its derivatives, which comprises oxidization of a hydrocarbon substrate in the presence of a homogeneous MC-type catalyst and an oxidant.

In the present invention, the homogeneous MC-type catalyst may be selected from Co/Br, Mn/Br, Co/Mn/Br and Co/Mn/M/Br, where M is selected from Ni, Fe, Zr, Hf, Zn, Ti, Cu, Cr, Na, K, Rb, Cs, Ta, Nb, Ta, Re, Ce, Pr, Nd, Mo, V and a combination thereof.

Preferably, the homogeneous MC-type catalyst is Co/ Mn/ Br or Co/Mn/M/Br.

More preferably, the homogeneous MC-type catalyst is Co/ Mn/ Br or Co/Mn/M/Br, where M is selected from K, Ni, Zr and a combination thereof.

As a target material of the present invention, i.e., as an organic acid or its derivatives, Examples herein disclose experimental results with regard to purified terephthalic acid ('PTA'). However, according to the researches of the present inventors, a process of the present invention may also be applied to various organic acids or derivatives thereof, thus showing superior effects. Further, it is obvious that one skilled in the art may also easily apply the present invention to various substrates other than PTA when referring to the experimental procedure and results as set forth in the following Examples. Therefore, as a target material of the present invention, "an organic acid or derivatives thereof" is not limited to PTA at all.

Representative Examples of the organic acid or derivatives thereof include but are not limited to PTA, IPA (isophthalic acid), TMA (trimellitic anhydride), TME (trimesic acid), PMDA (pyromellitic dianhydride), NDCA (2,6-naphthalene dicarboxylic acid), BPDA (biphenyl dianhydride), adipic acid, *p*-hydroxybenzoic acid, hydroxynaphthoic acid, 4,4'-dihydroxybiphenyl, BA (benzoic acid), PNBA (*p*-nitrobenzoic acid), PHBA (*p*-hydroxybenzoic acid), PABA (*p*-acetoxybenzoic acid), ONBA (o-nitrobenzoic acid), TBIA (5-t-butylisophthalic acid), PA (phthalic anhydride), CBP (4,4'-carboxybiphenyl), OBBA (4,4'-oxybisbenzoic acid), SBBA (4,4'-sulfonylbisbenzoic acid), CBBA (4,4'-carboxybisbenzoic acid), BTDA (benzophenone tetracarboxylic anhydride), OPAN (oxybisphthalic anhydride), SPAN (sulfonylbisphthalic anhydride), IPAN (isopropylidene bisphthalic anhydride), CPAN (4,4'-carboxybisphthalic anhydride), 6-FDA (hexafluoroisopropylidene diphthalic anhydride), 1,8-naphthoic anhydride, ANA (acetoxynaphthoic acid), PIDA (trimethylphenylindan dicarboxylic acid), PIDADA (trimethylphenylindan dianhydride), HCB (hexacarboxybenzene), CHDA (1,4-cyclohexanedicarboxylic acid), CHTA (cyclohexane tricarboxylic acid), DDA (2.6-decalin dicarboxylic acid), SDA(4,4'-stilbene dicarboxylic acid), 4-TFMPA (4-trifluoromethylphthalic acid), NA (nicotinic acid=3-carboxypyridine), indole-2-carboxylic acid, quinolinic acid, phthalate ester, diphenyl phthalate, diphenyl isophthalate, diphenyl terephthalate, dibenzyl terephthalate and a mixture thereof.

Preferably, the organic acid or its derivatives is PTA, IPA, TMA, TME, PMDA, NDCA, BPDA, adipic acid and a mixture thereof. More preferably, the organic acid or its derivatives is PTA, NDCA and a mixture thereof.

As used herein, an "oxidant" refers to a mixed gas of O₂/CO₂, and the volumetric ratio or partial pressure of O₂ in the mixed gas is preferably within the range of 30-40%, and more preferably 35-40%.

The present inventors have attempted to increase the partial pressure of O₂, unlike the conventional processes, which only disclose an oxidation under a relatively low partial pressure of O₂ in the premixed gas. According to the researches of the present inventors, as the partial pressure of O₂ increases to 30%, 35% and 40%, the oxidation of ρ-xylene undergoes a remarkable change in the reaction mechanism with an unexpectedly rapid progress, and that the effect levels off at the partial pressure of O₂ over 40%.

That is, when the volumetric ratio or partial pressure of O₂ is within the range of 30-40%, more preferably 35-40%, all the reaction results are improved such as oxidation rate, conversion, selectivity and yield of PTA, under even milder reaction conditions (e.g., reaction time, pressure and temperature).

All the commercial or known PTA processes produce a significant amount of impurities such as 4-CBA and p-toluic acid, and require a purification process for removing those impurities by hydrogenation reduction. In the present invention, the volumetric ratio or partial pressure of O₂ is increased to 30%-40%, thus unexpectedly causing a remarkable change in oxidation mechanism. As a result, the production of the impurities such as 4-CBA and p-toluic acid is much lowered even under milder conditions so that a complicated and high-cost purification process is not required, being another technical feature of the present invention.

Therefore, when the volumetric ratio or partial pressure of O₂ is lower than 30%, the production of impurities such as 4-CBA and p-toluic acid drastically increases, which necessitates additional purification process. The volumetric ratio or partial pressure of O₂ of higher than 40% is not preferred due to a rapid increase in danger caused by high O₂ pressure.

Further, the volumetric ratio or partial pressure of CO₂ in the O₂/CO₂ mixed gas is preferred to be within the range of 70-60%. Alternatively, the O₂/CO₂ mixed gas may further comprise 5-50% by volume of helium or argon.

However, the amount of N₂ in the O₂/CO₂ mixed gas is preferred to be reduced down to less than 20%, more preferably less than 5% by volume.

According to another embodiment of the present invention, the conversion and the production of color impurities such as benzyl, fluorenone and anthraquinone is preferably controlled by adjusting the reaction temperature.

Specifically, the oxidation process in the present invention is preferably conducted at 120-210°C, more preferably 130-190°C, most preferably 140-170°C.

When the reaction temperature is lower than the aforementioned lower limit, the conversion drastically decreases and the production of 4-CBA and p-toluic acid also remarkably increases. The reaction proceeds via a different reaction mechanism when the reaction temperature is higher than the aforementioned upper limits, and as a result, the production of the color impurities such as benzyl, fluorenone and anthraquinone increases, which cause the yellowish color of CTA.

According to an embodiment of the present invention, the production of the impurities, particularly the color impurities, is preferably controlled by varying the O₂ content in the mixed gas, the concentration of the catalyst and the reaction temperature. Although each substrate is preferred to undergo oxidation under a different reaction condition for maximizing the preferable effect of the present invention, one skilled in the art may easily find such reaction conditions such as a reaction temperature based on the disclosure herein and the experimental results below.

A process of the present invention also remarkably decreases the loss of acetic acid solvent, which is a serious problem in the commercial process.

According to another embodiment of the present invention, a process herein is preferably conducted by using a continuous stirred tank reactor system.

According to still another embodiment of the present invention, the impurities such as 4-CBA and p-toluic acid are preferred to remain in the amount of less than 25 ppm and less than 200 ppm, respectively, after the oxidation step.

As an unexpectedly remarkable effect of the present invention, total amount of color impurities in crude TA such as benzylbenzene, fluorenone and anthraquinone may be lowered down to a desired level only by conducting a simple process such as recrystallization or centrifugation without conducting a complicated and high-price purification process such as hydrogenation process.

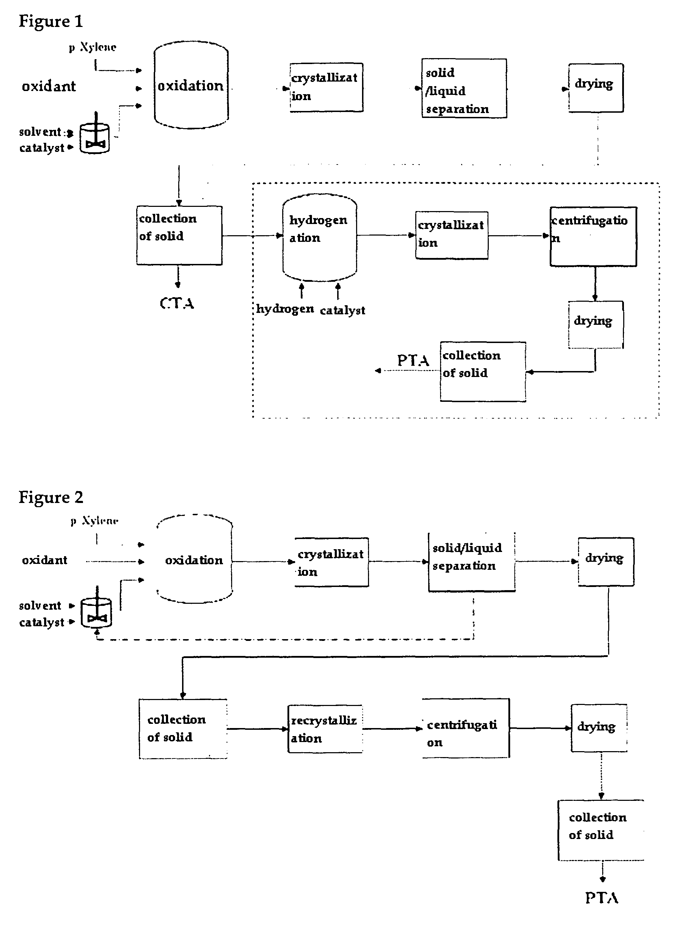

Further, although white color is preferred for the solid products produced by a process herein, light yellow color is also fine because this color turns into white by conducting the simple treatment of recrystallization or centrifugation instead of a complicated and high-cost purification process such as hydrogenation process (Figure 2).

According to a further embodiment of the present invention, carbon monoxide (CO) produced during the oxidation reaction herein is preferred to be transformed into carbon dioxide (CO₂) on an oxidation catalyst such as a commercial catalyst (Pt/C). The transformed carbon dioxide and reaction heat may be recycled for supplementation afterwards.

### BRIEF DESCRIPTION OF DRAWINGS

Figure 1 is a flow chart of a conventional process for preparing purified terephthalic acid, which comprises a reduction process (a hydrogenation treatment) for removing impurities such as 4-CBA in CTA.
Figure 2 is a flow chart of a process herein for preparing purified terephthalic acid, which only comprises simple processes such as recrystallization and centrifugation instead of other reduction processes such as hydrogenation process.

### EXAMPLES

The present invention is described more specifically by the following Examples. Examples herein are meant only to illustrate the present invention, but in no way to limit the scope of the claimed invention.

Various changes and modifications may be made in carrying out the present invention without departing from the spirit and scope thereof. Insofar as these changes and modifications are within the purview of the appended claims, they are to be considered as part of the present invention.

### Effect of partial pressure of O₂ in O₂/CO₂ mixed gas

### Example 1

Among various hydrocarbon substrates such as ρ-xylene, o-xylene, m-xylene 1,3,5-trimethylbenzene and 2,6-dimethylnaphthalene that may be oxidized into an organic acid according to the present invention, ρ-xylene was selected and oxidation was conducted as set forth in TABLE 2.

Specifically, acetic acid (glacial, 99.8%, Aldrich) was used as a solvent, and CoBr₂ (99%, Aldrich) and Mn(OAc)₂.4H₂O (99%, Aldrich) were used as catalyst. The catalyst was adjusted to contain 287 ppm of cobalt, 1,481 ppm of manganese and 779 ppm of bromine based on the weight of reactants.

The catalyst was admixed with 7 g of *p*-xylene, 107 g of acetic acid and 5 g of distilled water, thus providing 120g of the mixture. After the mixture was loaded into a 200 mL Ti-autoclave, oxidation was conducted at 170°C for one hour with stirring at 350 rpm, while introducing an O₂/CO₂ (30%/70%; 15 atm) mixed gas at a flow rate of 400 cc/min.

Products were subject to solid/liquid separation, and the liquid portion was analyzed with GC-MS (Agilent 5973I) and GC-FID (Agilent 6890N). The solid portion was dried and esterified, followed by the analysis by using GC-FID (Agilent 6890N).

As shown in TABLE 2, by-products such as 4-CBA and p-toluic acid were not detected, and the yield of terephthalic acid (referred to as 'TA' hereinafter) was 90.2%, which shows a remarkable increase in the yield of TA compared to Comparative Examples 1-3. From this result, it may be ascertained that the partial pressure of O₂ may be stably increased by using O₂/CO₂ mixed gas, and that the use of O₂/CO₂ mixed gas with relatively high partial pressure of O₂ may remarkably increase the conversion and the selectivity.

### Example 2

The experiment was conducted same as in Example 1 except that the volumetric ratio of O₂/CO₂ in the O₂/CO₂ mixed gas was changed to 35%/65%. The final products were also analyzed as described in Example 1.

The yield of TA was increased to 95.8%, and impurities such as 4-CBA and p-toluic acid were not detected. It was ascertained that the conversion and the selectivity are much improved as compared to Comparative Examples 1-3.

### Example 3

The experiment was conducted same as in Example 1 except that the volumetric ratio of O₂/CO₂ in the O₂/CO₂ mixed gas was changed to 40%/60%. The final products were also analyzed as described in Example 1.

The yield of TA was increased to 95.6%, and impurities such as 4-CBA were not detected. It was ascertained that the conversion and the selectivity are much improved as compared to Comparative Examples 1-3 and are equivalent to those in Example 2.

### Comparative Example 1

The experiment was conducted same as in Example 1 except that the volumetric ratio of O₂/CO₂ in the O_{2/}CO₂ mixed gas was changed to 21%/79%. The final products were also analyzed as described in Example 1.

The yield of TA was increased to 58.2%, and 2.7% of 4-CBA and 30.1% of p-toluic acid were observed. It was ascertained that the conversion and the selectivity are drastically lowered as compared to Examples 1-3.

### Comparative Example 2

The experiment was conducted same as in Example 1 except that the volumetric ratio of O₂/CO₂ in the O₂/CO₂ mixed gas was changed to 26%/74%. The final products were also analyzed as described in Example 1.

The yield of TA was increased to 67.1%, and 3.9% of 4-CBA and 21.2% of p-toluic acid were observed. It was ascertained that the conversion and the selectivity are drastically lowered as compared to Examples 1-3.

### Comparative Example 3

The experiment was conducted same as in Example 1 except that the volumetric ratio of O₂/CO₂ in the O₂/CO₂ mixed gas was changed to 28%/72%. The final products were also analyzed as described in Example 1.

The yield of TA was increased to 69.7%, and 2.5% of 4-CBA and 12.7% of p-toluic acid were observed. It was ascertained that the conversion and the selectivity are drastically lowered as compared to Examples 1-3.

**TABLE 2**

| | Temperature | O₂ | CO₂ | Yield (mol%) | | | Color^{d} |
|---|---|---|---|---|---|---|---|
| | | | | TA^{a} | *p*-TA^{b} | 4-CBA^{c} | |
| Ex.1 | 170 °C | 30% | 70% | 90.2 | 0 | 0 | Light yellow |
| Ex. 2 | 170 °C | 35% | 65% | 95.8 | 0 | 0 | Light yellow |
| Ex. 3 | 170 °C | 40% | 60% | 95.6 | 0 | 0 | Light yellow |
| Comp.Ex.1 | 170 °C | 21% | 79% | 58.2 | 30.1 | 2.7 | Light yellow |
| Comp. Ex. 2 | 170 °C | 26% | 74% | 67 | 21.2 | 3.9 | Light yellow |
| Comp. Ex. 3 | 170 °C | 28% | 72% | 69 | 12.7 | 2.5 | Light yellow |

| | | | | | | | |
|---|---|---|---|---|---|---|---|
| * a: terephthalic acid, b: p-toluic acid, c: 4-carboxybenzaldehyde, d: the color of the solid products | | | | | | | |

### Effect of oxidation temperature

### Example 4

The experiment was conducted same as in Example 1 except that the oxidation temperature was changed to 135 °C. The final products were also analyzed as described in Example 1.

As shown in TABLE 3, the yield of TA is 78.5%, and traces of impurities such as 4-CBA and p-toluic acid were observed, which were completely removed by increasing the reaction time to 3 hours. It was ascertained that the conversion is much improved as compared to Comparative Example 4. The color of the products (i.e. white) is also advantageous over Comparative Example 5 despite the relatively lower conversion and selectivity. The dark yellow color of solid products in Comparative Example 5 is due to large amount of color impurities such as 2,6-DCAq (2,6-dicarboxyanthraquinone).

### Example 5

The experiment was conducted same as in Example 1 except that the oxidation temperature was changed to 145 °C. The final products were also analyzed as described in Example 1.

The yield of TA is 89.7%, and traces of impurities such as 4-CBA and p-toluic acid were observed, which were completely removed by increasing the reaction time to 3 hours. It was ascertained that the conversion and the selectivity are much improved as compared to Comparative Example 4. The color of the products (i.e. white) is also advantageous over Comparative Example 5.

### Examples 6-8

The experiment was conducted same as in Example 1 except that the oxidation temperature was changed to 175 °C (Example 6), 185 °C (Example 7) and 195 °C (Example 8). The final products were also analyzed as described in Example 1.

The yields of TA are 92.4% (Example 6), 95.3% (Example 7) and 94.9% (Example 8), and impurities such as 4-CBA and p-toluic acid were not detected. It was ascertained that the conversion and the selectivity are much improved as compared to Comparative Example 4. Although the products are of yellow, the production of the color impurities such as 2,6-DCAq is negligible when compared to Comparative Example 5.

### Comparative Example 4

The experiment was conducted same as in Example 1 except that the oxidation temperature was changed to 115 °C. The final products were also analyzed as described in Example 1.

The yield of TA is 17.3%, and 2.6 % of 4-CBA and 29.3 % of p-toluic acid were observed. It was ascertained that the conversion and the selectivity are drastically lowered as compared to Examples 4-8, and that the color impurities were not completely removed although the reaction is conducted for 3 hours.

### Comparative Example 5

The experiment was conducted same as in Example 1 except that the oxidation temperature was changed to 220 °C. The final products were also analyzed as described in Example 1.

The yield of TA is 94.5%, and 4-CBA and *p*-toluic acid were not detected. The production of the color impurities such as 2,6-DCAq is higher than those of Examples 4-8. A large amount of loss in acetic acid was also observed due to a relatively higher reaction temperature.

**TABLE 3**

| | Temp. | O₂/CO₂ | Yield (mol%) Yield (mol%) | | | Color^{d} | 2,6-DCAq^{e} |
|---|---|---|---|---|---|---|---|
| | | | TA^{a} | *p*-TA^{b} | 4-CBA^{c} | | |
| Ex. 4 | 135 °C | 30%/70% | 78.5 | 8.4 | 1.7 | White | × |
| Ex. 5 | 145 °C | 30%/70% | 89.7 | 5.3 | 0.9 | White | × |
| Ex. 6 | 175 °C | 30%/70% | 92.4 | 0 | 0 | Light yellow | □ |
| Ex. 7 | 185 °C | 30%/70% | 95.3 | 0 | 0 | Yellow | ○ |
| Ex. 8 | 195 °C | 30%/70% | 94.9 | 0 | 0 | Yellow | ○ |
| Comp.Ex.4 | 115°C | 30%/70% | 17.3 | 29.3 | 2.6 | White | × |
| Comp.Ex.5 | 220 °C | 30%/70% | 94.5 | 0 | 0 | Dark yellow | ○○ |

| | | | | | | | |
|---|---|---|---|---|---|---|---|
| * a: terephthalic acid, b: p-toluic acid, c: 4-carboxybenzaldehyde, d: the color of the solid products, e: 2,6-dicarboxyanthraquinone (○○: large amount, ○: small amount, □ : trace, ×: not observed) | | | | | | | |

From the results above, it is ascertained that the production of impurities, particularly color impurities, may be controlled by varying O₂ content and temperature.

Further, although there is no experimental result disclosed in Examples herein, the present inventors also ascertained that each substrate should be treated under different oxidation conditions, particularly at different temperatures, for maximizing the preferable effects of the present invention. One skilled in the art may easily find such reaction conditions such as a reaction temperature based on the disclosure herein and the experimental results above.

### Effect of N₂ or Ar in O₂ in O₂/CO₂ mixed gas

### Example 9

The experiment was conducted same as in Example 1 except that the content of the O₂/CO₂mixed gas was changed to 30% of O₂, 40% of CO₂ and 30% of Ar. The final products were also analyzed as described in Example 1.

The yield of TA was 80.3%, and small amounts of impurities such as 4-CBA and p-toluic acid were observed, which were completely removed by increasing the reaction time to 1.5 hours. It was ascertained that the conversion is much improved as compared to Comparative Example 6, where N₂ was used instead of Ar. This result shows that the oxidation reaction is not inhibited by Ar but by N₂.

### Comparative Example 6

The experiment was conducted same as in Example 9 except that the content of the O₂/CO₂ mixed gas was changed to 30% of O₂, 40% of CO₂ and 30% of N₂. The final products were also analyzed as described in Example 1.

The yield of TA was 41.0%, and 1.8% of 4-CBA and 31.7% of p-toluic acid were observed, which were not completely removed although the reaction is conducted for 2 hours. This result shows that N₂ inhibits the oxidation reaction regardless of the reaction time.

### Comparative Example 7

The experiment was conducted same as in Example 9 except that the content of the O₂/CO₂ mixed gas was changed to 30 % of O₂ and 70 % of N₂. The final products were also analyzed as described in Example 1.

The yield of TA was 39.1%, and 4.0% of 4-CBA and 54.8% of p-toluic acid were observed, which were not completely removed although the reaction is conducted for 2 hours. In particular, 4-CBA was produced in a remarkably large amount as compared to Comparative Example 6.

**TABLE 4**

| | Temp. | Time | O₂ | CO₂ | Ar | N₂ | Yield (mol%) | | | Color^{d} |
|---|---|---|---|---|---|---|---|---|---|---|
| | | | | | | | TA^{a} | *p*-TA^{b} | 4-CBA^{c} | |
| Ex. 9 | 190 °C | 1 hr | 30% | 40% | 30% | - | 80.3 | 16.1 | 2.0 | Yellow |
| Comp. Ex. 6 | 190 °C | 1 hr | 30% | 40% | - | 30% | 41.0 | 31.7 | 1.8 | Yellow |
| Comp. Ex. 7 | 190 °C | 1 hr | 30% | - | - | 70% | 39.1 | 54.8 | 4.0 | Yellow |

| | | | | | | | | | | |
|---|---|---|---|---|---|---|---|---|---|---|
| *a: terephthalic acid, b: *p*-toluic acid, c: 4-carboxybenzaldehyde, d: the color of the solid products | | | | | | | | | | |

### Effect of oxidation time

### Example 10

The experiment was conducted same as in Example 1 except the followings: (i) the volumetric ratio of O₂/CO₂ in the O₂/CO₂ mixed gas was changed to 35%/65%, (ii) the oxidation temperature was changed to 190 °C and (iii) the oxidation time was changed to 50 minutes. The final products were also analyzed as described in Example 1.

As shown in TABLE 5, the yield of TA was 92.1 %, and 4-CBA and p-toluic acid were not detected, which were not completely removed although the reaction was conducted for 2 hours. Considering the results of Comparative Example 8, it was ascertained that the color impurities may be completely removed by a slight increase in the oxidation time.

### Comparative Example 8

The experiment was conducted same as in Example 10 except the oxidation time was changed to 40 minutes. The final products were also analyzed as described in Example 1.

As shown in TABLE 5, the yield of TA was 60.8 %, and 3.3% of 4-CBA and 19.1 % of p-toluic acid were observed.

### Comparative Example 9

The experiment was conducted same as in Example 10 except the volumetric ratio of O₂/CO₂ was changed to 30%/70% and that the oxidation time was changed to 40 minutes. The final products were also analyzed as described in Example 1.

As shown in TABLE 5, the yield of TA was 42.2%, and small amounts of 4-CBA and p-toluic acid were observed, which were completely removed by increasing the reaction time to 1 hour.

**TABLE 5**

| | Temp. | Time | O₂/CO₂ | Yield (mol%) | | | Color^{d} |
|---|---|---|---|---|---|---|---|
| | | | | TA^{a} | *p*-TA^{b} | 4-CBA^{c} | |
| Ex. 10 | 190 °C | 50 minutes | 35%/65% | 92.1 | 0 | 0 | Yellow |
| Comp. Ex. 8 | 190 °C | 40 minutes | 35%/65% | 68.0 | 19.1 | 3.3 | Yellow |
| Comp. Ex. 9 | 190 °C | 40 minutes | 30%/70% | 42.2 | 27.6 | 2.8 | Yellow |

| | | | | | | | |
|---|---|---|---|---|---|---|---|
| * a: terephthalic acid, b: p-toluic acid, c: 4-carboxybenzaldehyde, d: the color of the solid products | | | | | | | |

As described above, a process of the present invention leads to remarkable improvements in the oxidation rate, the conversion, the selectivity and the yield of PTA even under milder conditions. Further, the production of color impurities and other impurities such as 4-CBA and p-toluic acid may also be lowered down to a negligible level without hydrogenation reduction. Furthermore, the loss in acetic acid solvent due to combustion may also be drastically decreased according to the present invention.

## Claims

1. A process of preparing an organic acid or its derivatives in the presence of a homogeneous MC-type catalyst and an oxidant, comprising oxidizing a hydrocarbon substrate,
wherein the homogeneous MC-type catalyst is selected from the group consisting of Co/Br, Mn/Br, Co/Mn/Br and Co/Mn/M/Br wherein M is selected from the group consisting of Ni, Fe, Zr, Hf, Zn, Ti, Cu, Cr, Na, K, Rb, Cs, Ta, Nb, Ta, Re, Ce, Pr, Nd, Mo, V and a combination thereof;
the oxidant is an O₂/CO₂ mixed gas and the O₂ is contained 30-40% of the mixed gas in volume; and
the organic acid or its derivatives is selected from the group consisting of PTA (purified terephthalic acid), IPA (isophthalic acid), TMA (trimellitic anhydride), TME (trimesic acid), PMDA (pyromellitic dianhydride), NDCA (2,6-naphthalene dicarboxylic acid), BPDA (biphenyl dianhydride), adipic acid, *p*-hydroxylbenzoic acid, hydroxynaphthoic acid, 4,4'-dihydroxybiphenyl, BA (benzoic acid), PNBA (*p*-nitrobenzoic acid), PHBA (*p*-hydroxybenzoic acid), PABA (*p*-acetoxybenzoic acid), ONBA (o-nitrobenzoic acid), TBIA (5-t-butylisophthalic acid), PA (phthalic anhydride), CBP (4,4'-carboxybiphenyl), OBBA (4,4'-oxybisbenzoic acid), SBBA (4,4'-sulfonylbisbenzoic acid), CBBA (4,4'-carboxybisbenzoic acid), BTDA (benzophenone tetracarboxylic anhydride), OPAN (oxybisphthalic anhydride), SPAN (sulfonylbisphthalic anhydride), IPAN (isopropylidene bisphthalic anhydride), CPAN (4,4'-carboxybisphthalic anhydride), 6-FDA (hexafluoroisopropylidene diphthalic anhydride), 1,8-naphthoic anhydride, ANA (acetoxynaphthoic acid), PIDA (trimethylphenylindan dicarboxylic acid), PIDADA (trimethylphenylindan dianhydride), HCB (hexacarboxybenzene), CHDA (1,4-cyclohexane dicarboxylic acid), CHTA (cyclohexane tricarboxylic acid), DDA (2,6-decalin dicarboxylic acid), SDA (4,4'-stilbene dicarboxylic acid), 4-TFMPA (4-trivluoromethylphthalic acid), NA (nicotinic acid = 3-carboxypyridine), indole-2-carboxylic acid, quinolinic acid, phthalate ester, diphenyl phthalate, diphenyl isophthalate, diphenyl terephthalate, dibenzyl terephthalate and a mixture thereof.

2. A process of preparing an organic acid or derivatives thereof in the presence of a homogeneous MC-type catalyst and an oxidant, comprising oxidizing a hydrocarbon substrate;
wherein the homogeneous MC-type catalyst is selected from the group consisting of Co/Mn/Br and Co/Mn/M/Br (M is selected from the group consisting of K, Ni, Fe, Zr, Hf, Zn, Ti, Cu, Cr, Na, K, Rb, Cs, Ta, Nb, Ta, Re, Ce, Pr, Nd, Mo, V and a combination thereof);
the oxidant is an O₂/CO₂ mixed gas and the O₂ is contained 30-40% of the mixed gas in volume; and
the organic acid or its derivatives is selected from the group consisting of PTA (purified terephthalic acid), IPA (isophthalic acid), TMA (trimellitic anhydride), TME (trimesic acid), PMDA (pyromellitic dianhydride), NDCA (2,6-naphthalene dicarboxylic acid), BPDA (biphenyl dianhydride), adipic acid and a mixture thereof.

3. A process of preparing an organic acid or derivatives thereof in the presence of a homogeneous MC-type catalyst and an oxidant, comprising oxidization of a hydrocarbon substrate;
wherein the homogeneous MC-type catalyst is Co/Mn/Br or Co/Mn/M/Br (M is selected from the group consisting of Ni, Zr, K and a combination thereof);
the oxidant is an O₂/CO₂ mixed gas and the volumetric ratio of O₂ is 30-40%; and
the organic acid or derivatives thereof is selected from the group consisting of PTA (purified terephthalic acid), IPA (isophthalic acid), NDCA (2,6-naphthalene dicarboxylic acid) and a mixture thereof.

4. The process according to any of claims 1-3, wherein the oxidization is conducted at 120-210°C.

5. The process of claim 5, wherein the oxidization is conducted at 140-170°C.

6. The process according to any of claims 1-3, wherein the O₂ is contained 60-70% of the mixed gas in volume.

7. The process according to any of claims 1-3, wherein the O₂/CO₂ mixed gas comprises 5-50% by volume of helium or argon.

8. The process according to any of claims 1-3, wherein the O₂/CO₂ mixed gas comprises less than 20% by volume of nitrogen.

9. The process of claim 9, wherein the O₂/CO₂ mixed gas comprises less than 5% by volume of nitrogen.

10. The process according to any of claims 1-3, wherein the oxidization is conducted by using a continuous stirred tank reactor system.

11. The process according to any of claims 1-3, wherein the 4-CBA and the p-toluic acid are produced in the amounts of less than 25 ppm and less than 200 ppm, respectively, without conducting hydrogenation process.

12. The process of claim 9, wherein the organic acid or its derivatives is light yellow in color before conducting recrystallization or centrifugation, and it turns into white after the recrystallization or centrifugation.

13. The process according to any of claims 1-3, wherein carbon monoxide produced during the oxidation is transformed into carbon dioxide on an oxidation catalyst and the transformed carbon dioxide and reaction heat are recycled.
